(19) Europäisches Patentamt
European Patent Office
Office européen des brevets

(11) Veröffentlichungsnummer : **0 023 593**
**B1**

(12) # EUROPÄISCHE PATENTSCHRIFT

(45) Veröffentlichungstag der Patentschrift : **16.02.83**

(51) Int. Cl.³ : **C 07 D213/80**, C 07 D213/803, A 61 K 31/455

(21) Anmeldenummer : **80103895.1**

(22) Anmeldetag : **08.07.80**

(54) **Dihydronicotinsäurederivate und deren Herstellung sowie Verwendung.**

(30) Priorität : **13.07.79 CH 6555/79**

(43) Veröffentlichungstag der Anmeldung : **11.02.81 Patentblatt 81/06**

(45) Bekanntmachung des Hinweises auf die Patenterteilung : **16.02.83 Patentblatt 83/07**

(84) Benannte Vertragsstaaten : **AT BE CH DE FR GB IT LI LU NL SE**

(56) Entgegenhaltungen :
**EP A 0 001 041**
**EP A 0 003 144**

(73) Patentinhaber : **F. HOFFMANN-LA ROCHE & CO. Aktiengesellschaft**

**CH-4002 Basel (CH)**

(72) Erfinder : **Wick, Alexander Eduard, Dr.**
**10 Rue du Buisson Richard**
**F-78600 Le Mesnil-le Roy (FR)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 023 593 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Dihydronicotinsäurederivate und deren Herstellung sowie Verwendung

Die vorliegende Erfindung betrifft Dihydronicotinsäurederivate, insbesondere die neue 1-Aethyl-1,4-dihydro-6-(2-naphthyl)-4-oxonicotinsäure und deren Ester der Formel

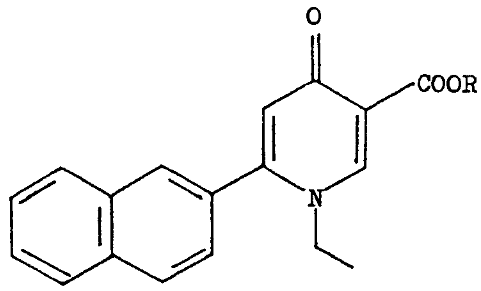

I

worin R Wasserstoff oder $C_{1-6}$-Alkyl darstellt, bzw. deren Alkalimetall-, Erdalkalimetall- und Ammoniumsalze und ein Verfahren zu deren Herstellung sowie deren Verwendung.

Als $C_{1-6}$-Alkylreste kommen gerad- oder verzweigtkettige Reste in Frage wie Methyl, Aethyl, Propyl, Isopropyl oder tert.-Butyl.

Die erfindungsgemässen Verbindungen können dadurch hergestellt werden, dass man die entsprechende 1,4,5,6-Tetrahydronicotinsäure bzw. deren Ester der Formel

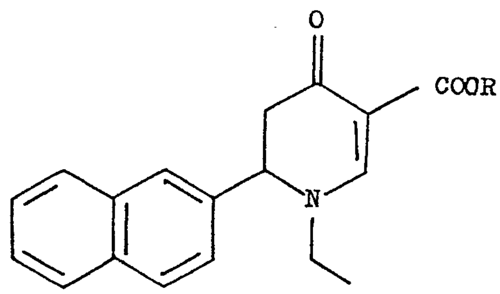

II

worin R die oben angegebene Bedeutung besitzt, in 5,6-Stellung dehydriert und gewünschtenfalls die erhaltene Säure in ein Alkalimetall-, Erdalkalimetall- oder Ammoniumsalz überführt oder mit einem $C_{1-6}$-Alkanol verestert, bzw. einen erhaltenen Methylester verseift.

Die erfindungsgemässe Dehydrierung einer Verbindung II kann nach an sich bekannten Methoden, zweckmässigerweise mit einem substituierten Benzochinon, wie 2,3-Dichlor-5,6-dicyan-1,4-benzochinon (DDQ) oder Tetrachlor-1,4-benzochinon (Chloranil), in einem inerten organischen Lösungsmittel, wie Methylenchlorid, Benzol oder Dioxan, und innerhalb eines Temperaturbereichs von Raumtemperatur bis zur Rückflusstemperatur des Reaktionsgemisches durchgeführt werden. Im allgemeinen geht man so vor, dass man zu der Lösung des Tetrahydronicotinsäurederivats eine Lösung des substituierten Benzochinons zutropft, wobei man darauf achtet, dass äquimolekulare Mengen umgesetzt werden. Bei ungefärbtem Ausgangsmaterial ist das Ende der Reaktion leicht durch die auftretende Färbung des Reaktionsgemisches zu erkennen. Das Reaktionsprodukt kann in üblicher Weise aus dem Gemisch isoliert und z. B. durch Umkristallisation oder chromatographische Verfahren gereinigt werden.

Sowohl die Veresterung einer Verbindung I mit R = Wasserstoff wie auch deren Ueberführung in die Salze bzw. die Verseifung einer Verbindung I mit R = Methyl zur Säure erfolgt in an sich bekannter Weise.

Die Ausgangsverbindungen der Formel II können gemäss dem folgenden Reaktionsschema aus 2-Naphthyl-aldehyd hergestellt werden.

Die Verbindungen der Formel I sind pharmakologisch aktiv. Sie weisen insbesondere eine stimulierende Wirkung auf das Zentralnervensystem auf, wobei sie durch geringe akute Toxizitäten gekennzeichnet sind. So ist beispielsweise 1-Aethyl-1,4-dihydro-6-(2-naphthyl)-4-oxonicotinsäure dem Nomifensin, bei etwa gleicher $LD_{50}$, wirkungsmässig wesentlich überlegen, während es sich vom d-Amphetamin und d-Methamphetamin bei etwa gleich starker Wirkung durch die wesentlich niedrigere $LD_{50}$ vorteilhaft abhebt (s. Tabelle I).

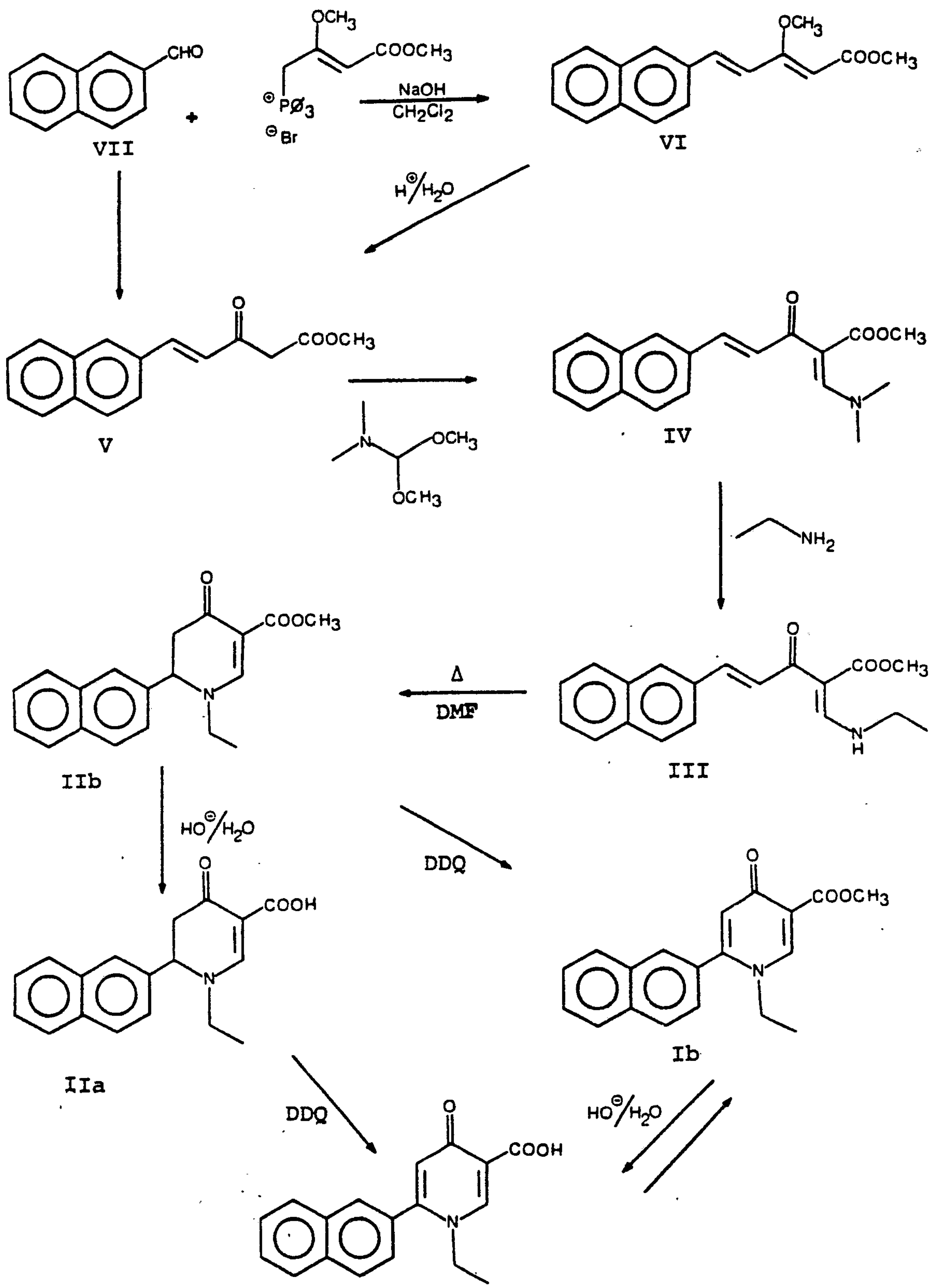


VII VI V IV III IIb IIa Ib Ia

Tabelle I

| Verbindung | $LD_{50}$ [mg/kg] (Maus) | Turning rat-Test [1] min. aktive Dosis [mg/kg] i. p. |
|---|---|---|
| 1-Aethyl-1,4-dihydro-6-(2-naphthyl)-4-oxonicotinsäure | 170 p. o. | 1 |
| Nomifensin | 300-600p. o. | 3 |
| d-Amphetamin · 1/2 $H_2SO_4$ | 35 p. o.<br>14 i. v.<br>22 s. c. | 1 |
| d-Methamphetamin · HCl | 9.5 i. v.<br>14 s. c. | 1 |

[1] Arch. int. Pharmacodyn. Ther. *217*, 118-130 (1975)

Die erfindungsgemässen Verbindungen der Formel I können daher zur Stimulierung des Zentralnervensystems in Form pharmazeutischer Präparate mit direkter oder verzögerter Freigabe des Wirkstoffes in Mischung mit einem für die orale oder parenterale Applikation geeigneten organischen oder anorganischen inerten Trägermaterial, z. B. Wasser, Gelatine, Gummi arabicum, Milchzucker, Stärke, Magnesiumstearat, Talk, pflanzlichen Oelen, Polyalkylenglykolen, Vaseline®, usw. verwendet werden. Die pharmazeutischen Präparate können in fester Form, z. B. als Tabletten, Dragées, Suppositorien, Kapseln ; in halbfester Form, z. B. als Salben ; oder in flüssiger Form, z. B. als Lösungen, Suspensionen oder Emulsionen, vorliegen. Gegebenenfalls sind sie sterilisiert und bzw. oder enthalten weitere Hilfsstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen.

Die Herstellung der pharmazeutischen Präparate kann in der jedem Fachmann geläufigen Weise, nämlich dadurch erfolgen, dass man die Wirksubstanz mit dem zur therapeutischen Verabreichung geeigneten, nichttoxischen, inerten Trägermaterial vermischt und das erhaltene Gemisch in die geeignete galenische Form bringt.

Als Dosierungsrichtlinie kann für die erfindungsgemässen Verbindungen eine Menge von 100 µg-10 mg/kg Körpergewicht pro Tag gelten.

Beispiel 1

12 g (38,8 mMol) Methyl-2-[(aethylamino)-methylen]-5-(2-naphthyl)-3-oxo-4-pentenoat wurden in 100 ml Dimethylformamid gelöst und während 4 Stunden bei 150 °C gerührt. Nach dem Abdampfen des Lösungsmittels unter Hochvakuum bei 60 °C erhielt man 12 g eines dunkelroten, zähen Oeles, das in 60 ml Toluol gelöst wurde. Man erhitzte zum Rückfluss und liess eine Aufschlämmung von 10,6 g (42,6 mMol) Chloranil in 50 ml Toluol langsam zutropfen. Das erhaltene Reaktionsgemisch wurde mit Essigester versetzt und 3mal mit eiskalter 3N Salzsäure ausgeschüttelt. Die sauren Auszüge wurden unter Kühlung mit 4N Natronlauge auf pH 6 gebracht und 3mal mit Methylenchlorid extrahiert. Die organischen Auszüge wurden mit gesättigter Kochsalz-Lösung gewaschen, über Natriumsulfat getrocknet und eingedampft. Man erhielt 10 g eines bräunlichen Oeles, das aus Aether/Methanol kristallisierte und 8,7 g (73 %) 1-Aethyl-1,4-dihydro-6-(2-naphthyl)-4-oxonicotinsäuremethylester, F. 138-139 °C (farblose Kristalle), lieferte.

3,8 g 1-Aethyl-1,4-dihydro-6-(2-naphthyl)-4-oxo-nicotinsäuremethylester wurden in 10 ml Methanol gelöst und mit 20 ml 1N Natronlauge versetzt. Nach 15 minütigem Rühren bei Raumtemperatur wurde das Reaktionsgemisch 2mal mit Essigester extrahiert und die wässrige Phase unter Kühlung mit 1N Salzsäure neutralisiert. Die dabei ausfallende freie Säure wurde abgesaugt, mit kaltem Wasser und Aether gewaschen und aus Aethanol/Methylenchlorid umkristallisiert. Man erhielt 3,1 g (85,5 %) 1-Aethyl-1,4-dihydro-6-(2-naphthyl)-4-oxo-nicotinsäure in Form farbloser Kristalle, F. 209-210 °C.

Das Ausgangsmaterial wurde folgendermassen erhalten :

Zu einer Lösung von 25 g (0,16 Mol) 1-Naphthylaldehyd und 78 g (0,165 Mol) 2-Methoxy-3-(methoxycarbonyl)-allyltriphenylphosphoniumbromid in 300 ml Methylenchlorid wurden unter Rühren bei Raumtemperatur 84 ml 50 %ige Natronlauge getropft. Nach 3stündigem Rühren bei Raumtemperatur und 1stündigem Rühren bei 40 °C wurden die Phasen getrennt. Die wässrige Phase wurde 2mal mit Methylenchlorid extrahiert und die vereinigten organischen Phasen wurden mit Wasser neutral gewaschen. Nach Trocknen über Natriumsulfat und Eindampfen des Lösungsmittels erhielt man ein viskoses, leicht gelbes Oel, das zum Teil kristallisierte. Das Gemisch wurde mit Aether/Essigester digeriert, eingedampft und aus Methanol umkristallisiert. Man erhielt 29,4 g (68,5 %) Methyl-3-methoxy-5-(2-naphthyl)-2,4-pentadienoat in Form farbloser Kristalle, F. 94-96 °C.

Eine Lösung von 23,5 g (87,7 mMol) Methyl-3-methoxy-5-(2-naphthyl)-2,4-pentadienoat in 500 ml Methanol wurde mit 50 ml konzentrierter Salzsäure versetzt. Nach ca. 3minütigem Rühren bei Raumtemperatur fiel ein weisses, kristallines Produkt aus, das abfiltriert, mit kaltem Wasser gewaschen und in Methylenchlorid gelöst wurde. Die organische Phase wurde 2mal mit eiskalter, gesättigter Natriumbicarbonat-Lösung ausgeschüttelt, über Natriumsulfat getrocknet und eingedampft. Nach Umkristallisation des kristallinen Rückstandes aus Methanol erhielt man 20,8 g (93 %) Methyl-5-(2-naphthyl)-3-oxo-4-pentenoat in Form farbloser Kristalle, F. 86-87 °C.

9 g (35,4 mMol) Methyl-5-(2-naphthyl)-3-oxo-4-pentenoat und 4,7 g (39 mMol) Dimethylformamiddimethyl-acetal wurden in 100 ml Toluol gelöst und während 22 Stunden bei 60 °C gerührt. Nach dem Abkühlen wurde das Reaktionsgemisch eingedampft. Man erhielt ca. 10 g eines dunkelroten, zähen Oeles, das mit einer Lösung von 100 g Aethylamin in 1 l Toluol versetzt wurde. Das Reaktionsgemisch wurde 20 Stunden bei Raumtemperatur gerührt, eingedampft und das Rohprodukt durch Säulenchromatographie [Kieselgel, Methylenchlorid/Methanol (19 : 1, v/v)] gereinigt. Nach dem Umkristallisieren aus Aether/Pentan erhielt man 6,0 g (55 %) Methyl-2-[(aethylamino)-methylen]-5-(2-naphthyl)-3-oxo-4-pentenoat in Form von leicht gelblichen Kristallen, F. 80-82 °C.

Beispiel 2

Es wurden Tabletten zu 120 mg bzw. 500 mg in an sich bekannter Weise hergestellt, enthaltend

| | | |
|---|---|---|
| 1-Aethyl-1,4-dihydro-6-(2-naphthyl)-4-oxonicotinsäure | 10,0 mg | 150 mg |
| Maisstärke | 50,0 mg | 160 mg |
| Milchzucker | 58,0 mg | 180 mg |
| Talk | 1,5 mg | 7 mg |
| Magnesiumstearat | 0,5 mg | 3 mg |
| | 120,0 mg | 500 mg |

**Ansprüche** (für die Vertragsstaaten : BE, CH, DE, FR, GB, IT, LI, LU, NL, SE)

1. 1-Aethyl-1,4-dihydro-6-(2-naphthyl)-4-oxonicotinsäure und deren Ester der Formel

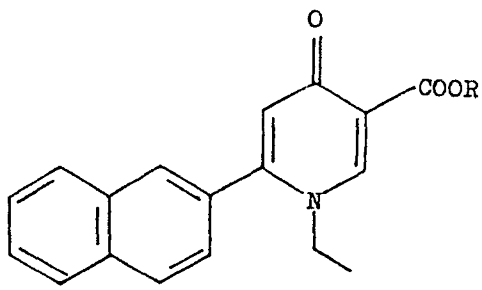

I

worin R Wasserstoff oder $C_{1-6}$-Alkyl darstellt, sowie deren Alkalimetall-, Erdalkalimetall- und Ammoniumsalze.

2. 1-Aethyl-1,4-dihydro-6-(2-naphthyl)-4-oxonicotinsäure.

3. 1-Aethyl-1,4-dihydro-6-(2-naphthyl)-4-oxonicotinsäuremethylester.

4. 1-Aethyl-1,4-dihydro-6-(2-naphthyl)-4-oxonicotinsäure und deren Ester gemäss einem der Ansprüche 1-3 zur Verwendung als pharmazeutische Wirkstoffe.

5. 1-Aethyl-1,4-dihydro-6-(2-naphthyl)-4-oxonicotinsäure und deren Ester gemäss einem der Ansprüche 1-3 zur Verwendung als Stoffe mit stimulierender Wirkung auf das Zentralnervensystem.

6. Verfahren zur Herstellung von 1-Aethyl-1,4-dihydro-6-(2-naphthyl)-4-oxonicotinsäure und deren Estern der Formel

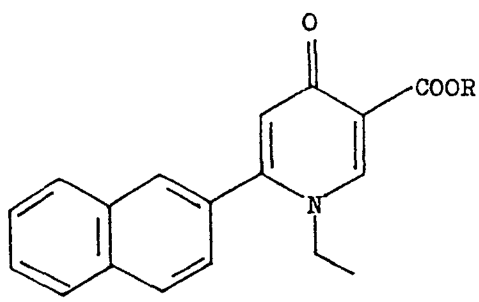

I

worin R Wasserstoff oder $C_{1-6}$-Alkyl darstellt, bzw. von deren Alkalimetall-, Erdalkalimetall- und Ammoniumsalzen, dadurch gekennzeichnet, dass man die entsprechende 1,4,5,6-Tetrahydronicotinsäure bzw. deren Ester der Formel

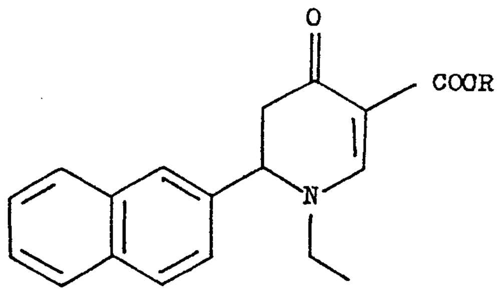

II

worin R die oben angegebene Bedeutung besitzt, in 5,6-Stellung dehydriert und gewünschtenfalls die erhaltene Verbindung in ein Alkalimetall-, Erdalkalimetall- oder Ammoniumsalz überführt oder mit einem $C_{1-6}$-Alkanol verestert, bzw. einen erhaltenen Methylester verseift.

7. Arzneimittel enthaltend eine Verbindung gemäss einem der Ansprüche 1-3.

8. Das Zentralnervensystem stimulierende Mittel enthaltend eine Verbindung gemäss einem der Ansprüche 1-3.

9. Verbindungen gemäss einem der Ansprüche 1-3 zur verwendung als Arzneimittel, insbesondere als das Zentralnervensystem stimulierende Mittel.

**Ansprüche** (für den Vertragsstaat AT)

1. Verfahren zur Herstellung von 1-Aethyl-1,4-dihydro-6-(2-naphthyl)-4-oxonicotinsäure und deren Estern der Formel

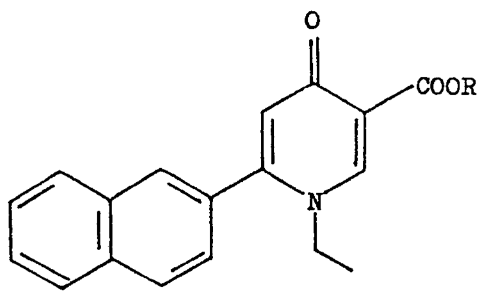

I

worin R Wasserstoff oder $C_{1-6}$-Alkyl darstellt, bzw. von deren Alkalimetall-, Erdalkalimetall- und Ammoniumsalzen, dadurch gekennzeichnet, dass man die entsprechende 1,4,5,6-Tetrahydronicotinsäure bzw. deren Ester der Formel

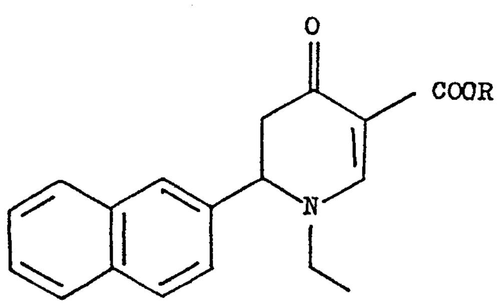

II

worin R die oben angegebene Bedeutung besitzt, in 5,6-Stellung dehydriert und gewünschtenfalls die erhaltene Verbindung in ein Alkalimetall-, Erdalkalimetall- oder Ammoniumsalz überführt oder mit einem $C_{1-6}$-Alkanol verestert, bzw. einen erhaltenen Methylester verseift.

2. Verfahren gemäss Anspruch 1, dadurch gekennzeichnet, dass man 1-Aethyl-1,4-dihydro-6-(2-naphthyl)-4-oxo-nicotinsäure herstellt.

3. Verfahren gemäss Anspruch 1, dadurch gekennzeichnet, dass man 1-Aethyl-1,4-dihydro-6-(2-naphthyl)-4-oxo-nicotinsäuremethylester herstellt.

**Claims** (for the Contracting States : BE, CH, DE, FR, GB, IT, LI, LU, NL, SE)

1. 1-Ethyl-1,4-dihydro-6-(2-naphthyl)-4-oxonicotinic acid and esters thereof of the formula

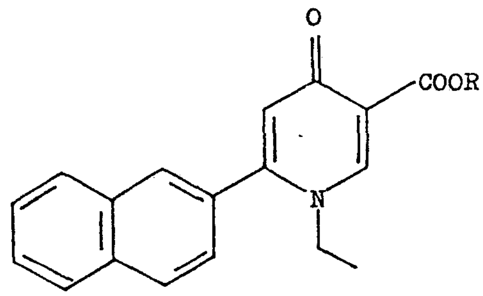

I

wherein R represents hydrogen or $C_{1-6}$-alkyl, as well as alkali metal, alkaline earth metal and ammonium salts thereof.

2. 1-Ethyl-1,4-dihydro-6-(2-naphthyl)-4-oxonicotinic acid.

3. 1-Ethyl-1,4-dihydro-6-(2-naphthyl)-4-oxonicotinic acid methyl ester.

4. 1-Ethyl-1,4-dihydro-6-(2-naphthyl)-4-oxonicotinic acid and esters thereof according to any one of claims 1-3 for use as pharmaceutically active substances.

5. 1-Ethyl-1,4-dihydro-6-(2-naphthyl)-4-oxonicotinic acid and esters thereof according to any one of claims 1-3 for use as substances with stimulating activity on the central nervous system.

6. Process for the manufacture of 1-ethyl-1,4-dihydro-6-(2-naphthyl)-4-oxonicotinic acid and esters thereof of the formula

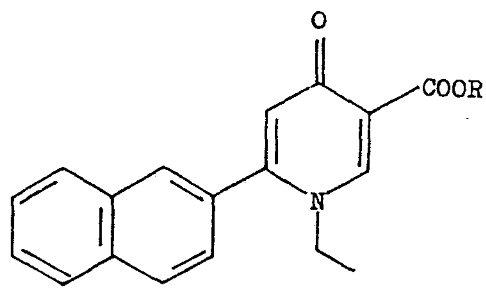

I

wherein R represents hydrogen or $C_{1-6}$-alkyl, or of alkali metal, alkaline earth metal and ammonium salts thereof, characterized in that the corresponding 1,4,5,6-tetrahydronicotinic acid or ester thereof of the formula

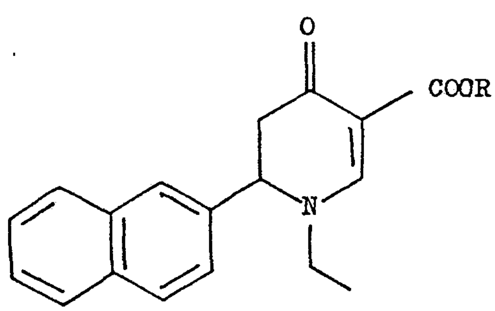

II

wherein R has the significance given above, is dehydrogenated in the 5,6-position and, if desired, the compound obtained is converted into an alkali metal, alkaline earth metal or ammonium salt or is esterified with a $C_{1-6}$-alkanol, or a methyl ester obtained is saponified.

7. Medicament containing a compound according to any one of claims 1-3.

8. The central nervous system stimulating medicament containing a compound according to any one of claims 1-3.

9. Compounds according to any one of claims 1-3 for use as medicaments, especially as central nervous system stimulating medicaments.

**Claims** (for the Contracting State AT)

1. Process for the manufacture of 1-ethyl-1,4-dihydro-6-(2-naphthyl)-4-oxonicotinic acid and esters thereof of the formula

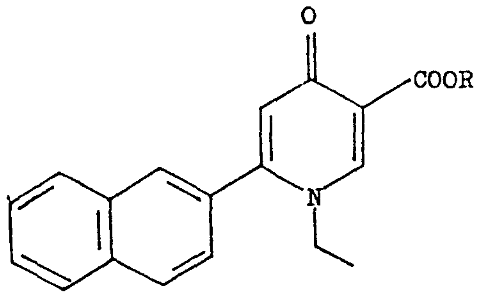

I

wherein R represents hydrogen or $C_{1-6}$-alkyl, or of alkali metal, alkaline earth metal and ammonium salts thereof, characterized in that the corresponding 1,4,5,6-tetrahydronicotinic acid or ester thereof of the formula

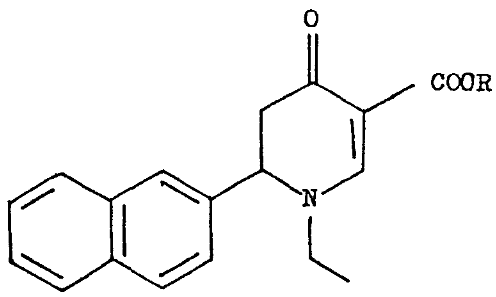

II

wherein R has the significance given above, is dehydrogenated in the 5,6-position and, if desired, the compound obtained is converted into an alkali metal, alkaline earth metal or ammonium salt or is esterified with a $C_{1-6}$-alkanol, or a methyl ester obtained is saponified.

2. Process according to claim 1 characterized in that 1-ethyl-1,4-dihydro-6-(2-naphthyl)-4-oxonicotinic acid is manufactured.

3. Process according to claim 1 characterized in that 1-ethyl-1,4-dihydro-6-(2-naphthyl)-4-oxonicotinic acid methyl ester is manufactured.

**Revendications** (pour les Etats contractants : BE, CH, DE, FR, GB, IT, LI, LU, NL, SE)

1. L'acide 1-éthyl-1,4-dihydro-6-(2-naphthyl)-4-oxonicotinique et des esters de formule

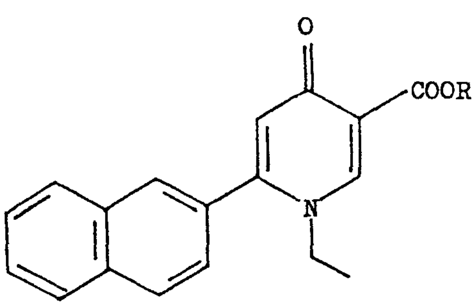

I

dans laquelle R représente l'hydrogène ou un groupe alkyle en C 1-C 6, ainsi que ses sels de métaux alcalins, de métaux alcalino-terreux et d'ammonium.

2. L'acide 1-éthyl-1,4-dihydro-6-(2-naphthyl)-4-oxonicotinique.

3. Le 1-éthyl-1,4-dihydro-6-(2-naphtyl)-4-oxonicotinate de méthyle.

4. L'acide 1-éthyl-1,4-dihydro-6-(2-naphthyl)-4-oxonicotinique et ses esters selon l'une des revendications 1 à 3 pour l'utilisation en tant que substances actives pharmaceutiques.

5. L'acide 1-éthyl-1,4-dihydro-6-(2-naphtyl)-4-oxonicotinique et ses esters selon l'une des revendications 1 à 3, pour l'utilisation en tant que substances possédant une activité stimulante sur le système nerveux central.

6. Procédé de préparation de l'acide 1-éthyl-1,4-dihydro-6-(2-naphthyl)-4-oxonicotinique et de ses esters de formule

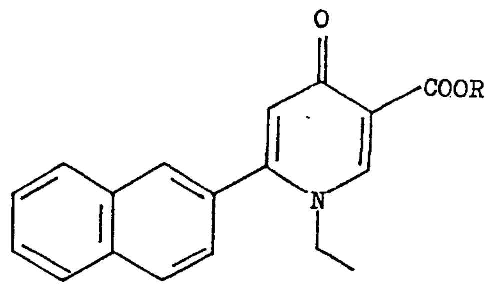

I

dans laquelle R représente l'hydrogène ou un groupe alkyle en C 1-C 6, et respectivement de ses sels de métaux alcalins, de métaux alcalino-terreux et d'ammonium, caractérisé en ce que l'on soumet l'acide 1,4,5,6-tétrahydro-nicotinique que ou respectivement ses esters de formule

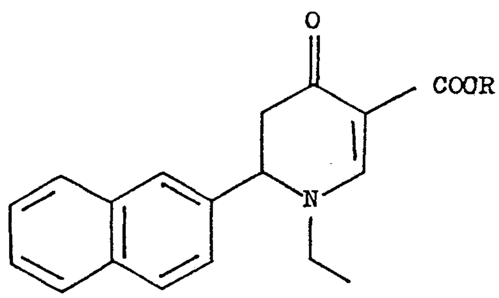

II

dans laquelle R a les significations indiquées ci-dessus, à déshydrogénation en position 5,6 et si on le désire, on convertit le composé obtenu en un sel de métal alcalin, de métal alcalino-terreux ou d'ammonium ou on l'estérifie par un alcanol en C 1-C 6, ou respectivement on saponifie l'ester méthylique obtenu.

7. Médicament contenant un composé selon l'une des revendications 1 à 3.

8. Produit stimulant le système nerveux central, contenant un composé selon l'une des revendications 1 à 3.

9. Composés selon l'une des revendications 1 à 3 pour l'utilisation en tant que médicaments, en particulier en tant qu'agents stimulant le système nerveux central.

**Revendications** (pour l'Etat contractant AT)

1. Procédé de préparation de l'acide 1-éthyl-1,4-dihydro-6-(2-naphthyl)-4-oxonicotinic et de ses esters de formule

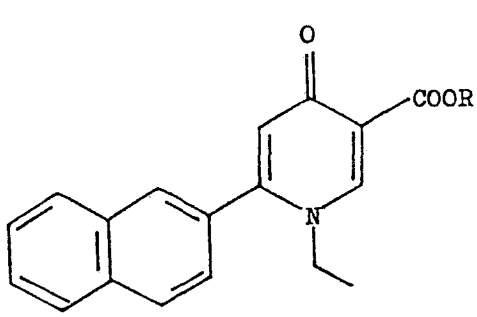

I

dans laquelle R représente l'hydrogène ou un groupe alkyle en C 1-C 6, et respectivement de ses sels de

métaux alcalins, de métaux alcalino-terreux et d'ammonium, caractérisé en ce que l'on soumet l'acide 1,4,5,6-tétrahydro-nicotinique ou respectivement ses esters de formule

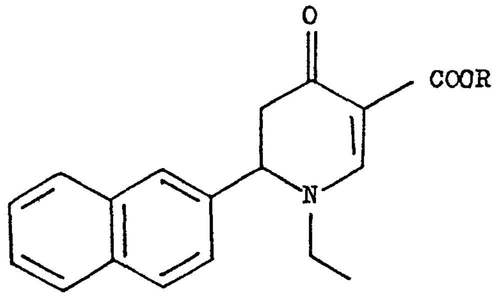

II

dans laquelle R a les significations indiquées ci-dessus, à déshydrogénation en position 5,6 et si on le désire, on convertit le composé obtenu en un sel de métal alcalin, de métal alcalino-terreux ou d'ammonium ou on l'estérifie par un alcanol en C 1-C 6, ou respectivement on saponifie l'ester méthylique obtenu.

2. Procédé selon la revendication 1 caractérisé en ce qu'on prépare l'acide 1-éthyl-1,4-dihydro-6-(2-naphtyl)-4-oxonicotinique.

3. Procédé selon la revendication 1 caractérisé en ce qu'on prépare le 1-éthyl-1,4-dihydro-6-(2-naphtyl)-4-oxonicotinate de méthyle.